(19) Europäisches Patentamt
European Patent Office
Office européen des brevets

(11) Veröffentlichungsnummer: **0 000 208**
**B1**

(12) **EUROPÄISCHE PATENTSCHRIFT**

(21) Anmeldenummer: **78200001.2**

(22) Anmeldetag: **01.06.78**

(51) Int. Cl³: **C 07 D 233/58**

# (54) Verfahren zur Herstellung von Imidazolen

(30) Priorität: **28.06.77 DE 2729017**
**28.06.77 DE 2728976**
**25.07.77 DE 2733466**

(43) Veröffentlichungstag der Anmeldung:
**10.01.79 Patentblatt 79/01**

(45) Bekanntmachung des Hinweises auf die Patenterteilung:
**30.04.80 Patentblatt 80/09**

(84) Benannte Vertragsstaaten:
**BE CH DE FR GB**

(56) Entgegenhaltungen:
**US - A - 2 226 057**
**US - A - 2 891 965**

(73) Patentinhaber: **BASF Aktiengesellschaft**
**Carl-Bosch-Strasse 38**
**D - 6700 Ludwigshafen (DE)**

(72) Erfinder: **Dockner, Toni, Dr.,**
**Grossgasse 6**
**D - 6701 Meckenheim (DE)**
**Frank, Anton**
**Bannwasserstrasse 72**
**D - 6700 Ludwigshafen (DE)**

EP 0 000 208 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von Imidazolen

Die Erfindung betrifft ein neues Verfahren zure Herstellung von Imidazolen durch Umsetzung von Carbonsäuren mit 1,2-Diaminen oder durch Umsetzung von 2-Imidazolinen bei Temperaturen von 300 bis 600°C in Gegenwart von Zinkoxid oder einem Gemisch von Zinkoxid und Aluminiumoxid als Katalysatoren.

Es ist aus der deutschen Offenlegungsschrift 1 952 991 bekannt, daß man Alkylendiamine mit Carbonylverbindungen in Gegenwart eines Kupfer- und/oder Chrom-Katalysators bei 320 bis 650°C zu Imidazolen umsetzen kann. Wie alle Beispiele zeigen, muß zusätzlich Wasserstoff der Reaktion zugeführt werden; wieder verwertbare Nebenprodukte werden nicht erhalten.

Die US-Patentschrift 2 847 417 beschreibt die Herstellung von Imidazolen aus Alkylendiaminen und Carbonsäuren an Trägerkatalysatoren der Platinmetalle. Solche Katalysatoren sind teuer und werden durch bestimmte Stoffe wie Schwefel, Schwermetalle oder Halogenide leicht vergiftet. Edelmetallkatalysatoren verlangen aufwendige Herstellungsverfahren, um die gleichmäßige Wirksamkeit des Katalysators zu gewährleisten, sowie sehr reine Ausgangsstoffe, um die Wirkung des Katalysators nicht zu beeinträchtigen. Wie Beschreibung (Spalte 1, Zeilen 50 bis 70, Spalte 2, Zeilen 65 bis 70) und die Beispiele zeigen, wird auch bei diesem Verfahren zusätzlich Wasserstoff zugesetzt, um die Bildung teeriger Polymere und Ablagerungen auf dem Katalysator zu vermeiden und die Wirksamkeit des Katalysators so möglichst lange zu erhalten.

Es ist aus der russischen Patentschrift 201 418 bekannt, daß man 2-Methylimidazolin in Gegenwart eines Metallkatalysators und mit Diphenyloxid als Reaktionsmedium bei einer Temperatur von 180 bis 230°C zu 2-Methylimidazol dehydriert. Die Patentschrift zeigt in einem Beispiel, daß Mengen von 47 Gramm Ausgangsstoff umgesetzt werden. Führt man die Umsetzung in der flüssigen Phase im großtechnischen Maße durch, erhält man eine wesentlich geringere Ausbeute bis zu weniger als 60 % der Theorie. Ein erheblicher Anteil an Imidazol wird durch Dealkylierung gebildet, und zwar um so mehr, je höher der Umsatz zu 2-Methylimidazol liegt. Das Verfahren ist daher im Hinblick auf Einsparung teuerer Lösungsmittel, gute Ausbeute und einfachen und wirtschaftlichen Betrieb unbefriedigend. Ein hoher Umsatz wäre andererseits erstrebenswert, weil so die destillative Abtrennung des unumgesetzten Imidazolins nicht mehr erforderlich ist. Ein solcher Vorteil wäre besonders im Falle der Herstellung hochschmelzender Imidazole, z.B. des 2-Phenylimidazols, von Bedeutung.

Es wurde nun gefunden, daß man Imidazole der Formel

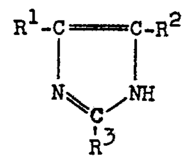

I

worin $R^1$, $R^2$, $R^3$ und $R^4$ gleich oder verschieden sein können und jeweils einen aliphatischen, araliphatischen, cycloaliphatischen oder aromatischen Rest oder ein Wasserstoffatom bedeuten, vorteilhaft erhält, wenn man

a) 1,2-Diamine der Formel

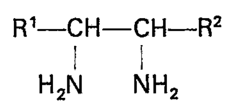

II

worin $R^1$ und $R^2$ die vorgenannte Bedeutung besitzen, mit Carbonsäuren der Formel

$$R^3—COOH \qquad III$$

worin $R^3$ die vorgenannte Bedeutung besitzt, oder

b) 2-Imidazoline der Formel

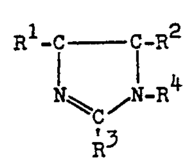

IV

worin $R^1$, $R^2$, $R^3$ und $R^4$ die vorgenannte Bedeutung besitzen, bei einer Temperatur von 300 bis 600°C

in Gegenwart von Zinkoxid oder einem Gemisch von Zinkoxid und Aluminiumoxid als Katalysatoren umsetzt.

Die Umsetzung kann für den Fall der Verwendung von 1,2-Diaminopropan und Propionsäure durch die folgenden Formeln wiedergegeben werden:

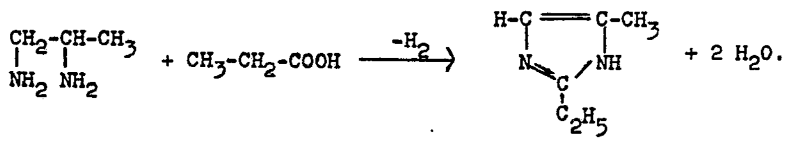


Die Umsetzung kann für den Fall der Verwendung von 1,2-Diphenylimidazolin durch die folgenden Formel wiedergegeben werden:

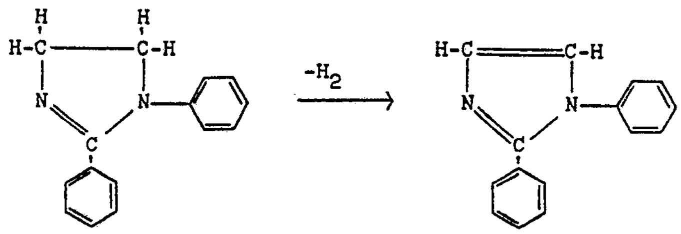


Im Vergleich zu den bekannten Verfahren liefert das Verfahren nach der Erfindung auf einfacherem und wirtschaftlicherem Wege Imidazole in guter Ausbeute und Reinheit. Obwohl kein zusätzlicher Wasserstoff zugeführt wird, werden eine Bildung teeriger Polymere, Ablagerungen auf dem Katalysator und ein rascher Rückgang der Katalysatoraktivität nicht beobachtet. Ein Zusatz von Wasserstoff ist nicht notwendig noch zweckmäßig. Im Vergleich zu den Katalysatoren der bekannten Verfahren sind die erfindungsgemäßen Katalysatoren billiger, leichter regenerierbar und werden nicht in deutlichem Maße während einer längeren Betriebsdauer vergiftet.

Gerade auch im großtechnischen Maßstab ist das Gesamtergebnis mit Bezug auf Umsatz und Ausbeute im Vergleich zum Stand der Technik besser.

Neben dem Endstoff fällt im Falle der Arbeitsweise a) als einziges wesentliches Nebenprodukt das dem Endstoff homologe Imidazolin an. Das Imidazolin ist für eine Reihe von Synthesen verwendbar oder kann zurückgeführt und nach einem Dehydrierungsverfahren, vorteilhaft der Verfahrensweise b) zum Endimidazol, dehydriert werden. Vergleichsweise zu den bekannten Verfahren liefert die Verfahrensweise a) nach der Erfindung somit eine wesentlich höhere Ausbeute an wieder verwertbaren Endprodukten bzw. an Endstoff I, bezogen auf dieselbe Menge Ausgangsstoff II. Alle diese vorteilhaften Eigenschaften sind im Hinblick auf den Stand der Technik überraschend.

Die Ausgangsstoffe II und III werden miteinander in stöchiometrischer Menge oder jeder im Überschuß zum anderen, vorteilhaft in einem Verhältnis von 1 bis 5, vorzugsweise von 1 bis 1,1 Mol Ausgangsstoff II je Mol Ausgangsstoff III umgesetzt. Bevorzugte Ausgangsstoffe II, III und IV und dementsprechend bevorzugte Endstoffe I sind solche, in deren Formeln $R^1$, $R^2$, $R^3$ und $R^4$ gleich oder verschieden sein können und jeweils einen Alkylrest mit 1 bis 18 Kohlenstoffatomen, insbesondere mit 1 bis 87 Kohlenstoffatomen, einen Alkenylrest mit mehreren oder insbesondere einer Doppelbindung und mit 2 bis 18, vorzugsweise 3 bis 18, insbesondere 4 bis 8 Kohlenstoffatomen, einen Aralkylrest mit 7 bis 12 Kohlenstoffatomen, einen Cycloalkylrest mit 5 bis 7 Kohlenstoffatomen oder einen Phenylrest oder ein Wasserstoffatom bedeuten. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein.

Als Ausgangsstoffe II kommen z.B. in Frage: Äthylendiamin, 1,2-Propylendiamin, 1,2-Butylendiamin, 1,2-Pentylendiamin, 1,2-n-Hexylendiamin, 1,2-n-Heptylendiamin, 1,2-n-Octylendiamin, 1,2-n-Nonylendiamin, 1,2-n-Decylendiamin, 1,2-n-Octadecylendiamin; 2,3-Butylendiamin, 2,3-Pentylendiamin, 2,3-Hexylendiamin, 2,3-Heptylendiamin, 2,3-Octylendiamin, 2,3-Nonylendiamin, 2,3-Decylendiamin, 3,4-Hexylendiamin, 3,4-Heptylendiamin, 3,4-Octylendiamin, 3,4-Nonylendiamin, 3,4-Decylendiamin, 4,5-Octylendiamin, 4,5-Nonylendiamin, 4,5-Decylendiamin, 5,6-Decylendiamin; durch die Benzyl- und/oder Phenylgruppe in 1-Stellung einfach oder in 1- und 2-Stellung gleichzeitig substituierte Äthylendiamine; durch vorgenannte Alkylgruppen in 1-Stellung und durch die Benzyl- oder Phenylgruppe in 2-Stellung substituierte Äthylendiamine.

Als Ausgangsstoffe III kommen z.B. in Betracht: Benzoesäure, Phenylessigsäure; Acrylsäure, Ameisensäure, Essigsäure, Propionsäure, Buttersäure, Isobuttersäure, Tetracosansäure, Hexacosansäure, Linolsäure, Linolensäure, Ricinolsäure, Erucasäure, Myristinsäure, Arachinsäure, Behensäure, Ölsäure, Elaidinsäure, Capronsäure, Önanthsäure, Pelargonsäure, Caprinsäure, 3,5,5-Trimethylhexansäure, 2-Äthylpenten-(2)-säure-(1), Undecansäure, Laurinsäure, Palmitinsäure, Stearinsäure, 2-Äthylhexancarbonsäure, $\alpha$-Äthylbuttersäure, Methacrylsäure, Crotonsäure, Isocrotonsäure,

Tiglinsäure, Sorbinsäure, Undecylensäure, 2-Methylbutansäure, Trimethylessigsäure, Valeriansäure, Isovaleriansäure, $\alpha$-Eläostearinsäure, $\Delta$-9,10-12,13-Octadecadiensäure, Isocapronsäure, Nonansäure, Tridecansäure, Pentadecansäure, Heptadecansäure, 4,5-Decensäure, 9,10-Decensäure, 4,5-Dodecensäure, 5,6-Tetradecensäure, 9,10-Hexadecensäure, 6,7-Octadecensäure, 9,10-Octadecensäure, 11,12-Octadecensäure, 3,4-Dodecensäure, Pariarsäure; oder entsprechende Gemische wie die bei der Herstellung von natürlichen oder synthetischen Fettsäuren erhaltenen Gemische. Solche Gemische fallen z.B. durch Fettspaltung, durch Paraffinoxidation oder durch die Oxosynthese aus Olefinen, Kohlenoxid und Wasser, an.

Als Ausgangsstoffe IV kommen z.B. in Frage: In 2-Stellung, 4-Stellung oder 5-Stellung einfach oder in 2 dieser Stellungen gleich oder unterschiedlich zweifach oder in diesen 3 Stellungen gleich oder unterschiedlich dreifach durch die Methyl-, Äthyl-, Allyl-, Crotyl-, Propyl-, Isopropyl-, Butyl-, Isobutyl-, Pentyl-, sek.-Butyl-, tert.-Butyl-, Hexyl-, Heptyl-, Octyl-, Oleyl-, n-Undecen-(11)-yl-(1)-, Nonyl-, Decyl-, Octadecyl-, Benzyl-, Phenylgruppe substituierte 2-Imidazoline, unsubstituiertes 2-Imidazolin; in 2-Stellung, 4-Stellung oder 5-Stellung einfach oder in 2 dieser Stellungen gleich oder unterschiedlich zweifach oder in diesen 3 Stellungen gleich oder unterschiedlich dreifach durch die Methyl-, Äthyl-, Allyl-, Crotyl-, Propyl-, Isopropyl-, Butyl-, Isobutyl-, Pentyl-, sek.-Butyl-, tert.-Butyl-, Hexyl-, Heptyl-, Octyl-, Oleyl-, n-Undecen-(11)-yl-(1)-, Nonyl-, Decyl-, Octadecyl-, Benzyl-, Cyclohexyl-, Cyclopentyl-, Toluyl-, Xylyl-, Naphthyl-, Methoxy-phenyl-, Äthoxyphenyl-, Äthylphenyl-, Dimethoxyphenyl-, Phenylgruppe substituierte 1-Phenyl-2-imidazoline, unsubstituiertes 1-Phenyl-2-imidazolin; homologe, in 1-Stellung durch die Methyl-, Äthyl-, Allyl-, Crotyl-, Propyl-, Isopropyl-, Butyl-, Isobutyl-, Pentyl-, sek.-Butyl-, tert.-Butyl-, Hexyl-, Heptyl-, Octyl-, Oleyl-, n-Undecen-(11)-yl-(1)-, Nonyl-, Decyl-, Octadecyl-, Cyclohexyl-, Cyclopentyl-, Benzyl-, Toluyl-, Xylyl-, Naphthyl-, Methoxyphenyl-, Äthoxyphenyl-, Äthylphenyl-, Dimethoxypehnylgruppe substituierte 2-Imidazoline.

Die Umsetzung wird bei einer Temperatur von 300 bis 600°C, zweckmäßig von 350 bis 500°C, im Falle der Arbeitsweise a) vorzugsweise von 400 bis 450°C, im Falle der Arbeitsweise b) vorzugsweise von 400 bis 480°C, drucklos oder unter Druck, im Falle der Arbeitsweise b) zweckmäßig von 1 bis 50 bar, kontinuierlich oder diskontinuierlich durchgeführt. In der Regel dient das Reaktionsgemisch auch als Lösungsmedium, gegebenenfalls können auch unter den Reaktionsbedingungen inerte organische, zweckmäßig mit Wasser kein Azeotrop bildende Lösungsmittel, z.B. aliphatische Kohlenwasserstoffe wie Petroläther oder Ligroin, verwendet werden. Unter den organischen Lösungsmitteln sind solche mit einem Siedepunkt über 120°C, zweckmäßig über 140°C, z.B. entsprechende Benzinfraktionen von 120 bis 160°C, bevorzugt.

Als Katalysator werden Zinkoxid allein oder ein Gemisch von Zinkoxid und Aluminiumoxid, zweckmäßig in einem Verhältnis von Zink zu Aluminium wie 1 bis 50, vorzugsweise 8 bis 10 Grammatom Zink je Grammaton Aluminium, und von 0,1 bis 1, vorzugsweise von 0,2 bis 0,4 Grammatom Zink je Mol Ausgangsstoff II oder IV verwendet. Als Aluminiumoxid kommen z.B. $\alpha$ und $\gamma$-Aluminiumoxid in Frage. Man kann auch Zinkverbindungen, die unter den Reaktionsbedingungen Zinkoxid ergeben, verwenden, z.B. ein mit Zinkchlorid oder Zinksulfat imprägniertes Aluminiumoxid. Ebenfalls kommen anstelle von Aluminiumoxid auch dieses Oxid enthaltende Stoffe oder Stoffgemische in Betracht, z.B. Aluminiumsilikat, Magnesiumaluminiumsilikathydrat, Dimagnesiumaluminiumsilikathydrat, Natriumaluminiumsilikat, Calciumaluminiumsilikat, Fullerde, Tone, Bleicherden wie Bentonit, Bauxit, Bimsstein, Andalusit, Kaolin, Allophane, Zeolithe, Mullit, Korund, $\gamma$-Tonerde, Hydrargillit, Böhmit.

Der Katalysator kann trägerfrei sein oder auch auf einem Träger, vorteilhaft in einer Menge von 1 bis 18 Gewichtsprozent Katalysator, bezogen auf den Träger, aufgebracht sein. Ebenfalls können vorgenannte Aluminiumverbindungen gleichzeitig in Gestalt des darin enthaltenen $Al_2O_3$ als Katalysatorkomponente und für das Zinkoxid als Träger dienen. Als Träger kommen zweckmäßig in Frage Kieselsäureverbindungen wie Silikate, z.B. Montmorillonit, Floridaerde, Quarz, Asbest; gefällte Kieselsäure, Kieselgel, Kieselgur; Titandioxid, Zirkondioxid, Zinndioxid, Aktivakohle; Erdalkalisulfate oder Erdalkaliphosphate, z.B. die Calsium- oder Bariumsalze; oder entsprechende Gemische vorgenannter Trägermaterialien. Die Herstellung der Trägerkatalysatoren wird nach den üblichen Verfahren, z.B. durch Auftragen der Zinkverbindung und gegebenenfalls der Aluminiumverbindung auf den Träger, Trocknen und Calcinieren, Beispielsweise zwischen 400 und 1 200°C in reduzierender, oxidierender oder inerter Atmosphäre, durchgeführt. Der Träger kann auch in seiner gewünschten geometrischen Form mit einer Lösung der Zinkverbindung allein bzw. der Zink- und Aluminiumverbindung, z.B. einer wäßrigen Lösung von Zinksulfat und gegebenenfalls Aluminiumsulfat, getränkt und getrocknet werden. Ebenfalls kann man das Trägermaterial mit der Zinkverbindung und gegenbenenfalls der Aluminiumverbindung und Wasser verkneten, in die gewünschte Form bringen, trocknen und bei einer Temperatur von 400 bis 1 200°C calcinieren.

Die Teilchengröße der Katalysatoren beträgt vorzugsweise von 0,05 bis 7, insbesondere 2 bis 4 Millimeter. Die Form kann beliebig, z.B. in Pillen-, Zylinder- oder Strangform, kugelförmig oder körnig, gewählt werden. Bevorzugt sind bei dem Katalysator auf Träger Porenvolumina von 0,05 bis 1 Milliliter je Gramm, spezifische Oberflächen von 1 bis 300 Quadratmeter je Gramm und Schüttgewichte von 0,4 bis 2,1 Gramm je Milliliter. Man kann auch mit Katalysator überzogene Rohre oder netzartige Träger verwenden.

Vorzugsweise werden die Katalysatoren auf dem Träger in Splitt- oder Kugelform in der Wirbelschicht

eingesetzt, wobei zweckmäßig Katalysatorteilchen mit Korngrößen von 0,005 bis 3 mm, insbesondere von 0,1 bis 1 mm, bevorzugt 0,2 bis 0,4 mm, verwendet werden. Die Schichthöhe des Katalysatorbettes im Wirbelzustand beträgt vorteilhaft 30 bis 2 000 Millimeter, im Falle der Arbeitsweise b) insbesondere 60 bis 80 Millimeter, oder wird zweckmäßig so gewählt, daß sich Verweilzeiten der Ausgangsstoffe II in der Katalysatorschicht von 0,01 bis 20, vorzugsweise von 5 bis 10 Sekunden ergeben. Bezüglich der Herstellung der Katalysatoren wird auf Houben-Weyl, Methoden der Organsichen Chemie, Band 4/2, Seiten 142 ff und Ullmanns Encyklopädie der technischen Chemie, Band 9, Seiten 271 ff, verwiesen.

Die Reaktion kann wie folgt durchgeführt werden: Die flüssigen oder zweckmäßig dampfförmigen Ausgangsstoffe II und III im Falle der Areitsweise a), zweckmäßig im Gemisch mit Inertgasen wie Stickstoff, werden bei der Reaktionstemperatur über den Katalysator bzw. Katalysator auf dem Träger in einem Festbett geleitet. Im Falle der Arbeitsweise b) wird der flüssige oder dampfförmige Ausgangsstoff IV, zweckmäßig im Gemisch mit Inertgasen, z.B. Stickstoff, bei der Reaktionstemperatur über den Katalysator bzw. Katalysator auf dem Träger in einem Festbett geleitet. Das aus dem Reaktor dampfförmig austretende Reaktionsgemisch wird gegebenenfalls dann in einem Zyklon entstaubt, in einer gekühlten Vorlage kondensiert. Durch fraktionierte Destillation wird zweckmäßig der Endstoff abgetrennt. Der Endstoff kann auch durch Umkristallisation oder Umfällung aus geeigneten Lösungsmitteln, z.B. mit Toluol, Dimethylformamid oder verdünnten Säuren, z.B. mit Ameisensäure, isoliert werden.

In einer bevorzugten Ausführungsform des Verfahrens werden die Ausgangsstoffe in einer Wirbelschicht bei der Reaktionstemperatur umgesetzt. Der Katalysator bzw. Katalysator auf Träger kann durch Inertgas, einem Gemisch von Ausgangsstoff II und III und Inertgas oder dem Ausgangsgemisch allein oder im Falle der Arbeitsweise b) einem Gemisch von Ausgangsstoff IV und Inertgas als Wirbelschichtgas bei Normaldruck oder vermindertem oder erhöhtem Druck in einer Wirbelschicht gehalten werden. Entsprechend kann die Gesamtmenge oder eine Teilmenge an Ausgangsstoff II und III oder eine Teilmenge an Ausgangsstoff IV getrennt von dem Wirbelschichtgas in den Wirbelschichtreaktor eingeleitet werden. Das Diamin II und die Carbonsäure III können im Falle der Arbeitsweise a) auch gemischt werden; die so gebildeten Salze werden zweckmäßig in einem beheizten Vorratsgefäß flüssig gehalten und in einen Verdampfer dosiert, der dem Wirbelschichtreaktor vorgeschaltet ist. Im Falle der Arbeitsweise b) kann Ausgangsstoff IV auch in einem beheizten Vorratsgefäß flüssig gehalten und in einen Verdampfer dosiert werden, der dem Wirbelschichtreaktor vorgeschaltet ist. Gleichzeitig leitet man vorteilhaft einen schwachen Stickstoffstrom, zweckmäßig von 5 000 bis 50 000 Volumenteilen Stickstoff je Stunde, durch den Verdampfer. Die verdampften Salze oder im Falle der Arbeitsweise b) der verdampfte Ausgangsstoff IV werden zusammen mit dem Stickstoffstrom durch das Katalysatorbett geleitet. Zwar ist Wasserstoff in allen diesen Fällen als Inertgas verwendbar, zweckmäßiger wird aber, schon aus Gründen der Wirtschaftlichkeit und Sicherheit, Stickstoff verwendet. Die Konzentration des Ausgangsstoffes IV im Inertgas beträgt vorteilhaft 0,1 bis 50 Volumenprozent. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 ff, verwiesen. Die Aufarbeitung des Reaktionsgemisches erfolgt in vorgenannter Weise.

In einer bevorzugten Ausführungsform werden 1,2-Diamin der Formel

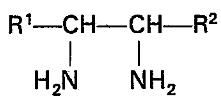
II

worin $R^1$ und $R^2$ die vorgenannte Bedeutung besitzen, mit Carbonsäuren der Formel

$$R^3—COOH \qquad III$$

worin $R^3$ die vorgenannte Bedeutung besitzt, bei einer Temperatur von 300 bis 600°C in Gegenwart von Zinkoxid oder einem Gemisch von Zinkoxid und Aluminiumoxid als Katalysatoren umgesetzt, wobei die als Nebenprodukte erhaltenen Imidazoline IV bei der fraktionierten Destillation anfallen und unter den vorgenannten Verfahrensbedingungen, z.B. mit Bezug auf Reaktionstemperatur, Katalysator, Reaktionsführung und Mengenverhältnissen der Komponenten, zu den entsprechenden Imidazolen dehydriert werden. Das Imidazolin wird vorteilhaft flüssig in den Verdampfer dosiert, die Dämpfe im Stickstoffstrom durch den Wirbelreaktor geleitet und anschließend kondensiert. Das Reaktionsgemisch kann ebenfalls durch Destillation oder Kristallisation gereinigt werden.

Die nach dem Verfahren der Erfindung herstellbaren Imidazole I sind wertvolle Ausgangsstoffe für die Herstellung von Farbstoffen, Pflanzenschutzmitteln, Textilhilfsmitteln, Katalysatoren für Polyurethane und Epoxidharze, oberflächenaktiven Mitteln und Pharmazeutika, z.B. den entsprechenden Nitroimidazolen. Imidazole I werden als Katalysatoren für Polymerisationsreaktionen und Aldolkondensationen verwendet. Bezüglich der Verwendung wird auf die verogenannten Veröffentlichungen und Ullmanns Encyklopädie der technischen Chemie, Band 8, Seite 499, verwiesen.

Die in den folgenden Beispielen aufgeführten Teile bedeuten Gewichtsteile. Die Gewichtsteile verhalten sich zu den Volumenteilen wie Kilogramm zu Liter.

Beispiel 1

120 Teile 1,2-Diaminoäthan werden unter Rühren und Kühlung mit 120 Teilen Essigsäure bei 38°C gemischt. Das gebildete Äthylendiamin-Monoacetat erstarrt unterhalb 35°C zu einer kristallinen Masse, oberhalb dieser Temperatur bleibt es flüssig. 200 Teile dieses Gemisches werden pro Stunde aus einem Vorratsgefäß in einen auf 300°C erhitzten horizontalen Quarz-Verdampfer dosiert und der Dampf zusammen mit 5 000 Volumenteilen pro Stunde Stickstoff durch den auf 350°C erhitzten Wirbelreaktor geleitet. Der Wirbelreaktor ist ein vertikal auf dem Verdampfer sitzendes, elektrisch beheiztes Quarzrohr, das nach unten mit einer eingeschmolzenen Quarzfritte abgeschlossen ist. Das Quarzrohr ist mit 200 Teilen eines Katalysators aus 90 Gewichtsprozent Zinkoxid und 10 Gewichtsprozent Aluminiumoxid (Krongröße 0,1 bis 0,3 mm) zur Hälfte gefüllt. Die Verweilzeit in der Katalysatorzone im Wirbelzustand beträgt 3,5 Sekunden. Die den Reaktor verlassenden Dämpfe werden kondensiert und fraktioniert destilliert. Man erhält stündlich 55,8 Teile (41 % der Theorie, bezogen auf umgesetztes Diamin II) 2-Methylimidazol vom $Kp_{15}$ 158°C, fp 145°C und 59,6 Teile (42,5 % der Theorie, bezogen auf umgesetztes Diamin II) 2-Methylimidazolin vom $Kp_{15}$ 115°C, Fp 103°C. Der Umsatz beträgt 98,7 Prozent, bezogen auf Diamin II. Die Ausbeute blieb auch noch nach 300 Stunden Betrieb konstant.

Beispiel 2

120 Teile Äthylendiamin und 92 Teile Ameisensäure werden stündlich bei 550°C zusammen mit 5 000 Volumenteilen Stickstoff durch den Wirbelreaktor geleitet. Man erhält analog Beispiel 1 stündlich 87,2 Teile (69,2 % der Theorie, bezogen auf umgesetztes Diamin II) Imidazol vom Fp 89°C.

Beispiel 3

90 Teile 1,2-Diaminoäthan, 110 Teile Propionsäure und 5 000 Volumenteile Stickstoff werden stündlich durch den auf 400°C erhitzten Wirbelschichtreaktor geleitet. Man erhält analog Beispiel 1 stündlich 67,2 Teile (47,3 % der Theorie, bezogen auf umgesetztes Diamin II) 2-Äthylimidazol vom Fp 78°C und 56,8 Teile (38,6 % der Theorie, benzogen auf umgesetztes Diamin II) 2-Äthylimidazolin vom $Kp_{17}$ 102°C. Der Umsatz beträgt 98,5 Prozent, bezogen auf Diamin II. Die Ausbeute blieb auch noch nach 300 Stunden Betrieb konstant.

Beispiel 4

74 Teile 1,2-Diaminopropan, 74 Teile Propionsäure und 10 000 Volumenteile Stickstoff werden stündlich durch den auf 380°C erhitzten Wirbelreaktor geleitet. Man erhält analog Beispiel 1 stündlich 7 Teile (6,2 % der Theorie, bezogen auf umgesetztes Diamin II) 2-Äthyl-4-methylimidazolin vom $Kp_{27}$ 111°C und 71,6 Teile (65,1 % der Theorie, bezogen auf umgesetztes Diamin II) 2-Äthyl-4-methylimidazol vom Fp 38°C. Der Umsatz beträgt 97,9 Prozent, bezogen auf Diamin II. Die Ausbeute blieb auch noch nach 300 Stunden Betrieb konstant.

Beispiel 5

Dem in Beispiel 1 beschriebenen Wirbelschichtreaktor werden aus zwei getrennten Vorratsgefäßen, die sich auf dem horizontalen Quarzverdampfer befinden, stündlich 100 Teile Essigsäure (Gefäß 1) und 100 Teile Äthylendiamin (Gefäß 2) zusammen mit 5 000 Volumenteilen Stickstoff zugeführt. Man erhält analog Beispiel 1 stündlich 71,3 Teile (52,1 % der Theorie, bezogen auf umgesetztes Diamin II) 2-Methylimidazol vom Fp 145°C. Der Umsatz beträgt 98,6 Prozent, bezogen auf Diamin II. Die Ausbeute blieb auch noch nach 300 Stunden Betrieb konstant.

Beispiel 6

Ein elektrisch beheizter Röhrenreaktor wird mit 10 mm langen und 4 mm dicken zylindrischen Füllörpern (200 Teile), bestehend aus 90 Gewichtsprozent Zinkoxid und 10 Gewichtsprozent Aluminiumoxid, gefüllt. Dieser Festbettreaktor sitzt senkrecht auf einem Quarzverdampfer. Stündlich werden 100 Teile 1,2-Diaminoäthan, 100 Teile Essigsäure im Quarzverdampfer bei 300°C verdampft und die Dämpfe zusammen mit 5 000 Volumenteilen/Stunde Stickstoff durch den auf 400°C erhitzten Reaktor geleitet. Man erhält analog Beispiel 1 stündlich 66,4 Teile (48,3 % der Theorie, bezogen auf umgesetztes Diamin II) 2-Methylimidazol vom Fp 145°C. Der Umsatz beträgt 97,9 %, bezogen auf Diamin II. Die Ausbeute blieb auch noch nach 300 Stunden Betrieb konstant.

Beispiel 7

a) 120 Teile 1,2-Diaminoäthan werden unter Rühren und Kühlung mit 120 Teilen Essigsäure bei 38°C gemischt. Das gebildete Äthylendiamin-Monoacetat erstarrt unterhalb 35°C zu einer kristallinen Masse, oberhalb dieser Temperatur bleibt es flüssig. 200 Teile dieses Gemisches werden pro Stunde aus einem Vorratsgefäß in einen auf 300°C erhitzten horizontalen Quarzverdampfer dosiert und der Dampf zusammen mit 5 000 Volumenteilen pro Stunde Stickstoff durch den auf 350°C erhitzten

Wirbelreaktor geleitet. Der Wirbelreaktor ist ein vertikal auf dem Verdampfer sitzendes, elektrisch, beheiztes, Quarzrohr, das nach unten mit einer eingeschmolzenen Quarzfritte abgeschlossen ist. Das Quarzrohr ist mit 200 Teilen eines Katalysators aus 90 Gewichtsprozent Zinkoxid und 10 Gewichtsprozent Aluminiumoxid (Korngröße 0,1 bis 0,3 mm) zur Hälfte gefüllt. Die Verweilzeit in der Katalysatorzone im Wirbelzustand beträgt 3,5 Sekunden. Die Höhe der Katalysatorzone beträgt im Wirbelzustand 80 mm. Die den Reaktor verlassenden Dämpfe werden kondensiert und fraktioniert destilliert. Man erhält stündlich 55,8 Teile (41 % der Theorie, bezogen auf umgesetztes Diamin II) 2-Methylimidazol vom $Kp_{15}$ 158°C und 59,6 Teile (42,5 Prozent der Theorie, bezogen auf umgesetztes Diamin II) 2-Methylimidazolin vom $Kp_{15}$ 115°C, Fp 103°C. Der Umsatz beträgt 98,7 Prozent, bezogen auf Diamin II. Die Ausbeute blieb auch noch nach 300 Stunden Betrieb konstant.

b) (Verwendung des Nebenproduktes): 200 Teile des nach Beispiel 7 a) erhaltenen 2-Methylimidazolins werden geschmolzen und stündlich mit 5 000 Volumenteilen $N_2$/Stunde bei 400°C durch den in Beispeil 7 a) beschriebenen Wirbelreaktor geleitet. Man erhält analog Beispiel 7 a) stündlich 121,3 Teile (91,1 % der Theorie, bezogen auf umgesetztes 2-Methylimidazolin) 2-Methylimidazol vom $Kp_{15}$ 158°C, Fp 145°C und 63,6 Teile nicht umgesetztes 2-Methylimidazolin vom $Kp_{15}$ 115°C, Fp 103°C. Umsatz = 68,2 Prozent.

Beispiel 8

Aus einem beheizten Dosiergsfäß werden stündlich 200 Teile geschmolzenes 2-Methylimidazolin in einen auf 300°C erhitzten Quarzverdampfer dosiert. Die Dämpfe werden analog Beispiel 7 a) zusammen mit 5 000 Volumenteilen $N_2$ durch den auf 400°C beheizten Wirbelschichtreaktor geleitet und nach Verlassen des Reaktors kondensiert. Man erhält analog Beispiel 7 b) stündlich 157,6 Teile 2-Methylimidazol vom Fp 145°C. Der Umsatz zu 2-Methylimidazol beträgt 87,15 Prozent, die Ausbeute 92,6 % der Theorie, bezogen auf umgesetztes 2-Methylimidazolin. Das nicht umgesetzte 2-Methylimidazolin wird durch fraktionierte Destillation abgetrennt und der Dehydrierung wieder zugeführt.

Beispiel 9

80 Teile 2-Phenylimidazolin und 100 000 Volumenteile Stickstoff werden stündlich durch den auf 400°C erhitzten Wirbelschichtreaktor geleitet. Man erhält analog Beispiel 8 stündlich 75 Teile (93,15 % der Theorie, bezogen auf umgesetzten Ausgangsstoff IV) 2-Phenylimidazol vom Fp 145°C. Der Umsatz beträgt 98 Prozent, bezogen auf umgesetzten Ausgangstoff IV. Die Ausbeute blieb auch noch nach 300 Stunden Betrieb konstant.

Beispiel 10

100 Teile 2-Äthyl-4-methylimidazolin und 1 000 Volumenteile Stickstoff werden stündlich durch den auf 380°C erhitzten Wirbelreaktor geleitet. Man erhält analog Beispiel 8 stündlich 71,25 Teile (91,5 % der Theorie, bezogen auf umgesetzten Ausgangsstoff IV) 2-Äthyl-4-methylimidazol vom Fp 38°C. Der Umsatz beträgt 79,3 Prozent, bezogen auf umgesetzten Ausgangsstoff IV. Die Ausbeute blieb auch noch nach 300 Stunden Betrieb konstant.

Beispiel 11

Analog Beispiel 7 werden stündlich 200 Teile 4-Methylimidazolin in einem Stickstoffstrom von 5 000 Teilen $N_2$/Stunde bei 400°C dehydriert. Man erhält analog Beispiel 8 stündlich 171 Teile (91,7 % der Theorie, bezogen auf umgesetztes Diamin II) 4-Methylimidazol vom Fp 45°C. Der Umsatz beträgt 95,5 Prozent, bezogen auf umgesetzten Ausgangsstoff IV. Die Ausbeute blieb auch noch nach 300 Stunden Betrieb konstant.

Beispiel 12

Analog Beispiel 8 werden stündlich 100 Teile 2-Heptadecylimidazolin in einem Stickstoffstrom von 5 000 Teilen $N_2$/Stunde bei 400°C dehydriert. Man erhält analog Beispiel 8 stündlich 88,9 Teile (98 % der Theorie, bezogen auf umgesetzten Ausgangsstoff IV) 2-Heptadecylimidazol vom $Kp_{0,4}$ 228°C, Fp 81°C neben 8,7 Teilen nicht umgesetztem Ausgangstoff IV. $Kp_{0,4}$ 194—197°C, Fp 77°C. Der Umsatz beträgt 91,3 Prozent.

Beispiel 13

Aus einem beheizten Dosiergefäß werden stündlich 100 Teile geschmolzenes 1-Phenylimidazolin in einen auf 300°C erhitzten, horizontalen Quarzverdampfer dosiert. Die Dämpfe werden zusammen mit stündlich 5 000 Volumenteilen $N_2$ durch einen auf 400°C beheizten Wirbelschichtreaktor geleitet. Der Wirbelreaktor ist ein vertikal auf dem Verdampfer sitzendes, elektrisch beheiztes Quarzrohr, das nach unten mit einer eingeschmolzenen Quarzfritte abgeschlossen ist. Das Quarzrohr ist mit 200 Teilen eines Katalysators aus 90 Gewichtsprozent Zinkoxid und 10 Gewichtsprozent Aluminiumoxid (Korngröß 0,1 bis 0,3 mm) zur Hälfte gefüllt. Die Verweilzeit in der Katalysatorzone im Wirbelzustand beträgt 3,5 Sekunden. Die Höhe der Katalysatorzone beträgt im Wirbelzustand 80 mm. Die den Reaktor verlassenden Dämpfe werden kondensiert und fraktioniert destilliert. Man erhält stündlich 69,5 Teile

(76 % der Theorie, bezogen auf umgesetzten Ausgangsstoff IV) 1-Phenylimidazol vom Kp (2 mbar) 110 bis 112°C neben 7,3 Teilen nicht umgesetztem 1-Phenylimidazolin vom Fp 45°C. Der Umsatz beträgt 92,7 Prozent, bezogen auf umgesetzten Ausgangsstoff IV. Die Ausbsute bleibt noch nach 72 Stunden Betrieb konstant.

Beispiel 14

100 Teile 1,2-Diphenylimidazolin und 50 000 Volumenteile Stickstoff werden stündlich durch den auf 550°C erhitzten Wirbelschichtreaktor geleitet. Man erhält analog Beispiel 13 stündlich 65 Teile (71,8 % der Theorie, bezogen auf umgesetzten Ausgangsstoff IV) 1,2-Diphenylimidazol vom Fp 90°C; der Umsatz beträgt 91,3 Prozent, bezogen auf eingesetzten Ausgangsstoff IV. Die Ausbsute bleibt noch nach 72 Stunden Betrieb konstant.

Beispiel 15

100 Teile 1-Phenyl-2-p-tolyl-imidazolin und 50 000 Volumenteile Stickstoff werden stündlich durch den auf 350°C erhitzten Wirbelschichtreaktor geleitet. Man erhält analog Beispiel 13 stündlich 70,6 Teile (81,3 % er Theorie, bezogen auf umgesetzten Ausgangstoff IV) 1-Phenyl-2-p-tolyl-imidazol vom Fp 119°C. Der Umsatz beträgt 87,6 %, bezogen auf eingesetzten Ausgangsstoff IV. Die Ausbeute bleibt noch nach 72 Stunden Betrieb konstant.

Beispiel 16

100 Teile 1-Phenyl-2-(3′,4′-dimethylphenyl)-imidazol und 50 000 Volumenteile Stickstoff werden stündlich durch den auf 400°C erhitzten Wirbelschichtreaktor geleitet. Man erhält analog Beispiel 13 stündlich 66 Teile (77,5 % der Theorie, bezogen auf umgesetzten Ausgangsstoff IV) 1-Phenyl-2-(3′,4′-dimethylphenyl)-imidazol vom Fp 146 bis 147°C (aus Äthylalkohol). Der Umsatz beträgt 85,9 Prozent, bezogen auf eingesetzten Ausgangstoff IV. Die Ausbeute bleibt noch nach 72 Stunden konstant.

Beispiel 17

100 Teile 1-Phenyl-2-cyclohexylimidazolin und 50 000 Volumenteile Stickstoff werden stündlich durch den auf 350°C erhitzten Wirbelschichtreaktor geleitet. Man fraktioniert den Reaktoraustrag über eine Kolonne und erhält beim $Kp_{0,1}$ 118°C 77,5 Teile 1-Phenyl-2-cyclohexylimidazol, entsprechend einer Ausbsute von 83,6 % der Theorie, bezogen auf umgesetzen Ausgangsstoff IV. Der Umsatz beträgt 93,6 %, bezogen auf eingesetzten Ausgangsstoff IV. Die Ausbeute bleibt noch nach 72 Stunden Betriebsdauer konstant.

**Patentanspruch**

Verfahren zur Herstellung von Imidazolen der Formel

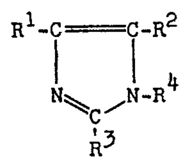

I

worin $R^1$, $R^2$, $R^3$ und $R^4$ gleich oder verschieden sein können und jeweils einen aliphatischen, araliphatischen, cycloaliphatischen oder aromatischen Rest oder ein Wasserstoffatome bedeuten, *dadurch gekennzeichnet*, daß man

a) 1,2-Diamine der Formel

$$\begin{array}{ccc} R^1—CH—CH—R^2 \\ \quad | \qquad\; | \\ \quad H_2N \quad NH_2 \end{array} \qquad II$$

worin $R^1$ und $R^2$ die vorgenannte Bedeutung besitzen, mit Carbonsäuren der Formel

$$R^3—COOH \qquad III$$

worin $R^3$ die vorgenannte Bedeutung besitzt, oder

b) 2-Imidazoline der Formel

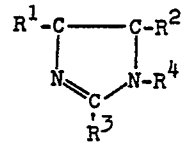 IV

worin $R^1$, $R^2$, $R^3$ und $R^4$ die vorgenannte Beteutung besitzen, bei einer Temperatur von 300 bis 600°C in Gegenwart von Zinkoxid oder einem Gemisch von Zinkoxid und Aluminiumoxid als Katalysatoren umsetzt.

**Claim**

A process for the production of imidazoles of the formula

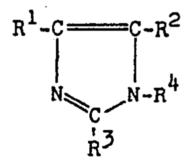 I

where $R^1$, $R^2$, $R^3$ and $R^4$ may be identical or different and each denotes an aliphatic, araliphatic, cycloaliphatic or aromatic radical or hydrogen, *characterized in that*

(a) 1,2-diamines of the formula

$$R^1-\underset{H_2N}{\underset{|}{CH}}-\underset{NH_2}{\underset{|}{CH}}-R^2 \qquad II$$

where $R^1$ and $R^2$ have the meanings given above are reacted with carboxylic acids of the formula

$$R^3-COOH \qquad III$$

where $R^3$ has the meaning given above, or

(b) 2-imidazolines of the formula

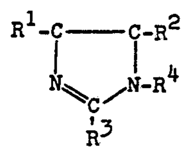 IV

where $R^1$, $R^2$, $R^3$ and $R^4$ have the meanings given above are reacted at from 300 to 600°C in the presence of zinc oxide or a mixture of zinc oxide and aluminium oxide as catalyst.

**Revendication**

Procédé pour la préparation d'imidazoles de formule

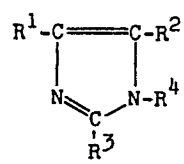 (I)

dans laquelle $R^1$, $R^2$, $R^3$ et $R^4$ peuvent être semblables ou différents et représentent chacun un radical aliphatique, araliphatique, cycloaliphatique ou aromatique, ou un atom d'hydrogène, caractérisé en ce qu'on fait réagir, à une température de 300 à 600°C, en présence d'oxyde de zinc ou d'un mélange d'oxyde de zinc et d'oxyde d'aluminium servant de catalyseur:

a) des 1,2-diamines de formule

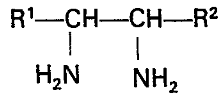

(II)

dans laquelle $R^1$ et $R^2$ ont les significations données ci-dessus, avec des acides carboxyliques de formule

$$R^3—COOH \qquad (III)$$

dans laquelle $R^3$ a la signification donnée ci-dessus, ou

b) des 2-imidazolines de formule

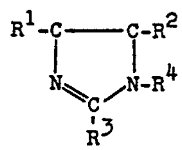

(IV)

dans laquelle $R^1$, $R^2$, $R^3$ et $R^4$ ont les significations données ci-dessus.